# EUROPEAN PATENT APPLICATION

(11) **EP 4 338 954 A1**
(43) Date of publication of application: **20.03.2024**
(21) Application number: 22820179.4
(22) Date of filing: 06.06.2022
(51) Int. Cl.: B32B 27/30, C08F 8/12, C08F 18/08, C09D 5/16, C09D 131/04, C12M 3/00

(54) **COMPOSITION FOR FORMING COATING FILM, COATING FILM AND CELL CULTURE CONTAINER**

(30) Priority: 07.06.2021 JP 2021095130
(71) Applicant: Nissan Chemical Corporation, Tokyo 103-6119 (JP)
(72) Inventor: HIROI, Miya, Funabashi-shi, Chiba 274-0052 (JP); HIROI, Yoshiomi, Funabashi-shi, Chiba 274-0052 (JP); UEDA, Yuki, Funabashi-shi, Chiba 274-0052 (JP); SUZUKI, Kohei, Funabashi-shi, Chiba 274-0052 (JP); FUKASAWA, Natsuki, Shiraoka-shi, Saitama 349-0294 (JP)
(74) Representative: dompatent von Kreisler Selting Werner - Partnerschaft von Patent- und Rechtsanwälten mbB
(86) International application number: PCT/JP2022/022743
(87) International publication number: WO 2022/259998

(57) **Abstract**

Provided is a coating film which is obtained by applying a composition for forming a coating film, the composition containing a copolymer, and which is used to suppress adhesion of a biological substance, in which the copolymer is water-insoluble, the copolymer contains a repeating unit (A) represented by the following Formula (A) and a repeating unit (B) represented by the following Formula (B), and a molar ratio (A : B) of the repeating unit (A) to the repeating unit (B) in the copolymer is 89 : 11 to 50 : 50. 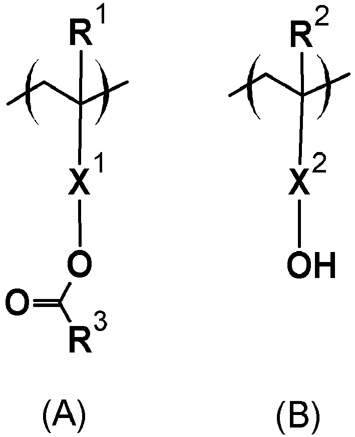 (In the formula, R¹ to R³ each independently represent a hydrogen atom or an alkyl group having 1 to 5 carbon atoms, and X¹ and X² each independently represent a single bond, an ester bond, an ether bond, an amide bond, or an alkylene group having 1 to 5 carbon atoms which may be interrupted by an oxygen atom.)

## Description

### Technical Field

The present invention relates to a coating film and a composition for forming a coating film that are used for suppressing adhesion of a biological substance, and a substrate with a coating film and a cell culture vessel using the same.

### Background Art

In order to suppress adhesion of a biological substance to various medical devices such as an artificial dialyzer, an artificial organ, a medical device, a cell culture vessel, and a substrate for cell culture, coating materials having an ability to suppress adhesion of various biological substances have been proposed. Patent Literature 1 discloses an ion complex material having an ability to control adhesion of a biological substance. Patent Literature 2 discloses a culture vessel for human ES cells.

### Citation List

### Patent Literature

Patent Literature 1: WO 2014/196650 A
Patent Literature 2: WO 2013/183777 A

### Summary of Invention

### Technical Problem

An object of the present invention is to provide a coating film that can suppress adhesion of a biological substance and is hardly dissolved in phosphate buffered saline, a composition for forming a coating film that is capable of forming a coating film that can suppress adhesion of a biological substance and is hardly dissolved in phosphate buffered saline, and a substrate with a coating film and a cell culture vessel using the same.

### Solution to Problem

As a result of intensive studies to achieve the above object, the present inventors have found that a coating film that can suppress adhesion of a biological substance and is hardly dissolved in phosphate buffered saline is obtained by using a specific copolymer, thereby completing the present invention.

[1] A coating film which is obtained by applying a composition for forming a coating film, the composition containing a copolymer, and which is used to suppress adhesion of a biological substance,
   in which the copolymer is water-insoluble,
   the copolymer contains a repeating unit (A) represented by the following Formula (A) and a repeating unit (B) represented by the following Formula (B), and
   a molar ratio (A : B) of the repeating unit (A) to the repeating unit (B) in the copolymer is 89 : 11 to 50 : 50. 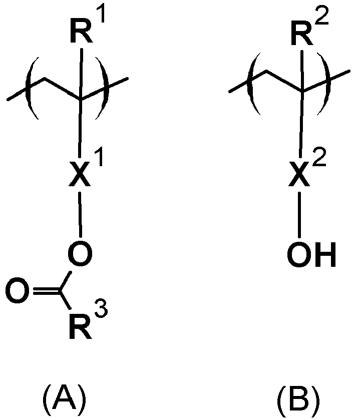
   (In the formula, R¹ to R³ each independently represent a hydrogen atom or an alkyl group having 1 to 5 carbon atoms, and X¹ and X² each independently represent a single bond, an ester bond, an ether bond, an amide bond, or an alkylene group having 1 to 5 carbon atoms which may be interrupted by an oxygen atom.)
[2] The coating film according to [1], in which R¹ and R² are hydrogen atoms, R³ is a methyl group, and X¹ and X² are single bonds.
[3] The coating film according to [1] or [2], in which a viscosity-average polymerization degree of the copolymer is 200 to 3,000.
[4] The coating film according to any one of [1] to [3], in which a total mol% of the repeating unit (A) and the repeating unit (B) in all the repeating units in the copolymer is 99.5 mol% or more.
[5] The coating film according to any one of [1] to [4], in which the copolymer does not contain an azide group.
[6] The coating film according to any one of [1] to [5], in which the copolymer is not crosslinked in the coating film.
[7] A composition for forming a coating film which is used to suppress adhesion of a biological substance, the composition containing a copolymer,
   in which the copolymer is water-insoluble,
   the copolymer contains a repeating unit (A) represented by the following Formula (A) and a repeating unit (B) represented by the following Formula (B), and
   a molar ratio (A : B) of the repeating unit (A) to the repeating unit (B) in the copolymer is 89 : 11 to 50 : 50. 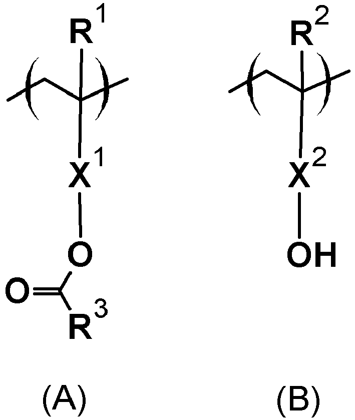
   (In the formula, R¹ to R³ each independently represent a hydrogen atom or an alkyl group having 1 to 5 carbon atoms, and X¹ and X² each independently represent a single bond, an ester bond, an ether bond, an amide bond, or an alkylene group having 1 to 5 carbon atoms which may be interrupted by an oxygen atom.)
[8] The composition for forming a coating film according to [7], in which R¹ and R² are hydrogen atoms, R³ is a methyl group, and X¹ and X² are single bonds.
[9] The composition for forming a coating film according to [7] or [8], in which a viscosity-average polymerization degree of the copolymer is 200 to 3,000.
[10] The composition for forming a coating film according to any one of [7] to [9], in which a total mol% of the repeating unit (A) and the repeating unit (B) in all the repeating units in the copolymer is 99.5 mol% or more.
[11] The composition for forming a coating film according to any one of [7] to [10], in which the copolymer does not contain an azide group.
[12] The composition for forming a coating film according to any one of [7] to [11], further containing a solvent.
[13] The composition for forming a coating film according to [12], in which the solvent contains an alcohol.
[14] A coating film which is obtained by applying the composition for forming a coating film according to any one of [7] to [13] and is used to suppress adhesion of a biological substance.
[15] The coating film according to [14], in which the copolymer is not crosslinked in the coating film.
[16] The coating film according to any one of [1] to [6], [14], and [15], in which a thickness of the coating film is 1 to 2,000 nm.
[17] A substrate with a coating film, including a substrate, and the coating film according to any one of [1] to [6] and [14] to [16] disposed on the substrate.
[18] A cell culture vessel including the substrate with a coating film according to [17].
[19] A method for manufacturing a coating film, the method including the steps of:
   supplying the composition for forming a coating film according to any one of [7] to [13] onto a substrate; and
   drying the composition for forming a coating film supplied onto the substrate.
[20] The method for manufacturing a coating film according to [19], in which the copolymer is not crosslinked.

### Advantageous Effects of Invention

According to the present invention, it is possible to provide a coating film that can suppress adhesion of a biological substance and is hardly dissolved in phosphate buffered saline, a composition for forming a coating film that is capable of forming a coating film that can suppress adhesion of a biological substance and is hardly dissolved in phosphate buffered saline, and a substrate with a coating film and a cell culture vessel using the same.

### Brief Description of Drawings

Fig. 1 is a photograph of a microscopic observation result of a cell adhesion test in Example 1.
Fig. 2A is a photograph of a microscopic observation result of a cell aggregate formation test in Example 3 (2 hours after seeding).
Fig. 2B is a photograph of a microscopic observation result of a cell aggregate formation test in Example 3 (one day after seeding).
Fig. 3 is a photograph of a microscopic observation result of a cell adhesion test in Comparative Example 3.
Fig. 4 is a photograph of a microscopic observation result of a cell adhesion test in Example 28.
Fig. 5A is a photograph of a microscopic observation result of a cell aggregate formation test in Example 28 (2 hours after seeding).
Fig. 5B is a photograph of a microscopic observation result of a cell aggregate formation test in Example 28 (one day after seeding).
Fig. 6 is a photograph of a microscopic observation result of a spheroid formation experiment in Example 31.
Fig. 7 is a photograph of a microscopic observation result of a spheroid formation experiment in Comparative Example 7.

### Description of Embodiments

### (Composition for forming coating film)

A composition for forming a coating film of the present invention is used to suppress adhesion of a biological substance.

The composition for forming a coating film contains at least a copolymer, and further contains other components such as a solvent as necessary.

Note that the composition for forming a coating film of the present invention can form a coating film that is hardly dissolved in phosphate buffered saline, but the application of the composition for forming a coating film of the present invention is not particularly limited as long as it is used to suppress adhesion of a biological substance, and is not limited to formation of a coating film in contact with phosphate buffered saline.

### <Copolymer>

The copolymer is water-insoluble. Here, the term "water-soluble" means that 1.0 g or more of the copolymer can be dissolved in 100 g of water at 25°C. The term "water-insoluble" means that the copolymer does not correspond to "water-soluble", that is, the solubility in 100 g of water at 25°C is less than 1.0 g.

The copolymer contains a repeating unit (A) represented by the following Formula (A) and a repeating unit (B) represented by the following Formula (B).

A molar ratio (A : B) of the repeating unit (A) to the repeating unit (B) in the copolymer is 89 : 11 to 50 : 50.

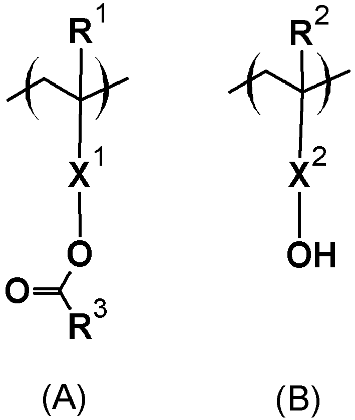

(In the formula, R¹ to R³ each independently represent a hydrogen atom or an alkyl group having 1 to 5 carbon atoms, and X¹ and X² each independently represent a single bond, an ester bond, an ether bond, an amide bond, or an alkylene group having 1 to 5 carbon atoms which may be interrupted by an oxygen atom.)

The copolymer may contain two or more kinds of repeating units (A).

The copolymer may contain two or more kinds of repeating units (B).

The copolymer preferably contains one kind of repeating unit (A) and one kind of repeating unit (B).

Examples of an alkyl group having 1 to 5 carbon atoms include a methyl group, an ethyl group, an n-propyl group, an isopropyl group, an n-butyl group, an isobutyl group, an s-butyl group, a t-butyl group, an n-pentyl group, a 1-methylbutyl group, a 2-methylbutyl group, a 3-methylbutyl group, a 1,1-dimethylpropyl group, a 1,2-dimethylpropyl group, a 2,2-dimethylpropyl group, and a 1-ethylpropyl group.

R¹ to R³ are each independently preferably a hydrogen atom, a methyl group, or an ethyl group.

The "ester bond" means -C(=O)-O- or -O-C(=O)-, the "ether bond" means -O-, and the "amide bond" means - NHC(=O)- or -C(=O)NH-.

An alkylene group having 1 to 5 carbon atoms may be interrupted by an oxygen atom.

Examples of the alkylene group having 1 to 5 carbon atoms include a methylene group, an ethylene group, a propylene group, a trimethylene group, a tetramethylene group, a 1-methylpropylene group, a 2-methylpropylene group, a dimethylethylene group, an ethylethylene group, a pentamethylene group, a 1-methyl-tetramethylene group, a 2-methyl-tetramethylene group, a 1,1-dimethyl-trimethylene group, a 1,2-dimethyl-trimethylene group, a 2,2-dimethyl-trimethylene group, and a 1-ethyl-trimethylene group. X¹ and X² are preferably methylene groups, ethylene groups, or propylene groups.

The phrase "may be interrupted by an oxygen atom" means that one or two or more carbon-carbon bonds of an alkylene group having 1 to 5 carbon atoms are bonded via an ether bond.

The copolymer is preferably a copolymer in which R¹ and R² are hydrogen atoms, R³ is a methyl group, and X¹ and X² are single bonds.

A molar ratio (A : B) of the repeating unit (A) to the repeating unit (B) is 89 : 11 to 50 : 50.

When the total number of moles of the repeating unit (A) and the repeating unit (B) in the copolymer is 100, the molar ratio (A : B) of the repeating unit (A) to the repeating unit (B) can be represented by (100-m) : m. In the case, a range of m is 11 to 50. A lower limit of m may be 12, 13, 14, 15, 16, 17, 18, 19, 20, 21, 22, 23, 24, 25, 26, 27, 28, 29, or 30. An upper limit of m may be 49, 48, 47, 46, 45, 44, 43, 42, 41, 40, 38, 37, 36, or 35. The range of m is, for example, 12 to 49, 12 to 48, 15 to 48, 20 to 49, 20 to 45, 22 to 49, or 22 to 45.

The total mol% of the repeating unit (A) and the repeating unit (B) in all the repeating units in the copolymer is not particularly limited, and is preferably 90 mol% or more, more preferably 95 mol% or more, still more preferably 99.5 mol% or more, and particularly preferably 100%.

In the present invention, the molar ratio of the repeating unit (A) to the repeating unit (B) in the copolymer is set to be within a specific range in order to obtain a coating film that is hardly dissolved in phosphate buffered saline. Therefore, in the present invention, a coating film that is hardly dissolved in phosphate buffered saline can be obtained without crosslinking of the copolymer. Therefore, the copolymer does not need to contain a photosensitive group for crosslinking the copolymer. That is, it is preferable that the copolymer does not contain a photosensitive group. Examples of the photosensitive group include an azide group.

In the present invention, the copolymer does not need to contain a photosensitive group for crosslinking the copolymer. Therefore, it is not required to perform light irradiation for crosslinking the copolymer when forming a coating film. Accordingly, a process of forming a coating film can be simplified.

A viscosity-average polymerization degree (hereinafter, also referred to as a "polymerization degree") of the copolymer is not particularly limited, and is preferably 200 to 3,000, more preferably 200 to 2,500, and particularly preferably 200 to 2,000, from the viewpoint of preferably obtaining the effect of the present invention.

The viscosity-average polymerization degree is measured in a state where the copolymer is completely saponified.

The "viscosity-average polymerization degree" of polyvinyl alcohol obtained by complete saponification is a value calculated by the following equation from a limiting viscosity [η] (g/dL) measured at 30°C by an Ostwald viscometer using ion-exchanged water as a solvent. $log \left(P\right) = 1.613 \times log \left(\left[η\right]\times {10}^{4}/8.29\right)$

Here, P represents a viscosity-average polymerization degree.

The viscosity-average polymerization degree can be determined according to JIS K 6726.

A method for producing a copolymer is not particularly limited, and examples thereof include a method in which a compound represented by the following Formula (C) is polymerized to produce a homopolymer, and the obtained homopolymer is partially hydrolyzed by a known saponification reaction to obtain a copolymer. 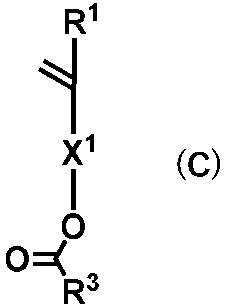 (In the formula, R¹, R³, and X¹ have the same meaning as described above.)

In addition, examples of a method for producing a copolymer include a method of copolymerizing a compound represented by the following Formula (C) and a compound represented by the following Formula (D) to obtain a copolymer. 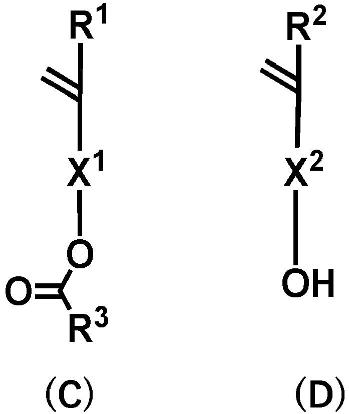 (In the formula, R¹ to R³, X¹, and X² have the same meaning as described above.)

The copolymer may be a random copolymer or may be a block copolymer.

As the copolymer, a commercially available product may be used. Specific examples of the commercially available product of the copolymer include polyvinyl acetate (manufactured by JAPAN VAM & POVAL CO., LTD., trade name: JMR-10L (registered trademark)).

A content of the copolymer in a film-forming component in the composition for forming a coating film is not particularly limited, and is preferably 80 mass% or more, more preferably 90 mass% or more, and particularly preferably 95 mass% or more. Note that the film-forming component refers to a component obtained by removing a solvent component from all the components of the composition.

A content of the copolymer in the composition for forming a coating film is not particularly limited, and is preferably 0.1 to 10 mass%, more preferably 0.3 to 8 mass%, and particularly preferably 0.5 to 5 mass%, from the viewpoint of easily forming a coating film having a desired thickness. In addition, the content of the copolymer in the composition for forming a coating film may be 0.02 to 2 mass% or may be 0.05 to 1 mass%.

### <Solvent>

Examples of the solvent include water, phosphate buffered saline (PBS), an alcohol, and a water-soluble organic solvent (but, an alcohol is excluded).

Examples of the alcohol include an alcohol having 2 to 6 carbon atoms.

Examples of the alcohol include ethanol, propanol, isopropanol, 1-butanol, 2-butanol, isobutanol, t-butanol, 1-pentanol, 2-pentanol, 3-pentanol, 1-heptanol, 2-heptanol, 2,2-dimethyl-1-propanol (neopentyl alcohol), 2-methyl-1-propanol, 2-methyl-1-butanol, 2-methyl-2-butanol (t-amyl alcohol), 3-methyl-1-butanol, 3-methyl-3-pentanol, cyclopentanol, 1-hexanol, 2-hexanol, 3-hexanol, 2,3-dimethyl-2-butanol, 3,3-dimethyl-1-butanol, 3,3-dimethyl-2-butanol, 2-ethyl-1-butanol, 2-methyl-1-pentanol, 2-methyl-2-pentanol, 2-methyl-3-pentanol, 3-methyl-1-pentanol, 3-methyl-2-pentanol, 3-methyl-3-pentanol, 4-methyl-1-pentanol, 4-methyl-2-pentanol, 4-methyl-3-pentanol, and cyclohexanol. These alcohols can be used alone or in combination of two or more kinds thereof.

The water-soluble organic solvent refers to an organic solvent that can be mixed with water and an alcohol at an arbitrary ratio, and does not cause separation after mixing.

Examples of the water-soluble organic solvent include ethylene glycol monomethyl ether, ethylene glycol monoethyl ether, methyl cellosolve acetate, ethyl cellosolve acetate, diethylene glycol monomethyl ether, diethylene glycol monoethyl ether, propylene glycol, propylene glycol monomethyl ether, propylene glycol monoethyl ether, propylene glycol monomethyl ether acetate, and propylene glycol propyl ether acetate. These water-soluble organic solvents can be used alone or in combination of two or more kinds thereof.

In the composition for forming a coating film, water, phosphate buffered saline (PBS), an alcohol, or a water-soluble organic solvent may be used alone as a solvent.

In the composition for forming a coating film, a combination of two or more of water, phosphate buffered saline (PBS), an alcohol, and a water-soluble organic solvent may be used as a solvent.

From the viewpoint of the solubility of the copolymer, the solvent is preferably selected from water, an alcohol, a water-soluble organic solvent, and a combination of two or more thereof, and is more preferably selected from water, ethanol, a water-soluble organic solvent, and a combination of two or more thereof.

As the combination of solvents, the following combinations are preferable.
· Water and alcohol
· Water, alcohol, and water-soluble organic solvent
· Alcohol and water-soluble organic solvent

As the combination of solvents, the following combinations are more preferable.
· Water and ethanol
· Water, ethanol, and propylene glycol monomethyl ether
· Ethanol and propylene glycol monomethyl ether

A mass ratio of water : alcohol in the composition for forming a coating film is, for example, 1 : 99 to 70 : 30 or 1 : 99 to 50 : 50.

A mass ratio (A : B : C) of water : alcohol : water-soluble organic solvent in the composition for forming a coating film is, for example, 5 to 30 : 65 to 92 : 1 to 30 (here, A+B+C is 100).

A mixing ratio (mass ratio) of alcohol : water-soluble organic solvent in the composition for forming a coating film is, for example, 30 : 70 to 97 : 3.

A content of the solvent in the composition for forming a coating film is not particularly limited, and is preferably 90 mass% or more, more preferably 92 mass% or more, and particularly preferably 95 mass% or more, from the viewpoint of easily forming a coating film having a desired thickness.

### <Other components>

The composition forming a coating film can contain other components as necessary.

Examples of the other components include a pH adjusting agent, a preservative, a surfactant, an antifungal agent, and saccharides.

### <Biological substance>

In the present invention, examples of the biological substance include a protein, a sugar, a virus, a nucleic acid, a cell, and a combination of two or more thereof.

Examples of the protein include fibrinogen, bovine serum albumin (BSA), human albumin, various kinds of globulin, β-lipoprotein, various kinds of antibodies (IgG, IgA, and IgM), peroxidase, various kinds of complements, various kinds of lectins, fibronectin, lysozyme, von Willebrand factor (vWF), serum γ-globulin, pepsin, ovalbumin, insulin, histone, ribonuclease, collagen, and cytochrome c.

Examples of the sugar include glucose, galactose, mannose, fructose, heparin, and hyaluronic acid.

Examples of the nucleic acid include deoxyribonucleic acid (DNA) and ribonucleic acid (RNA).

Examples of the cell include fibroblast, myeloid cell, B lymphocyte, T lymphocyte, neutrophil, erythrocyte, platelet, macrophage, monocyte, osteocyte, pericyte, dendritic cell, keratinocyte, adipocyte, mesenchymal cell, epithelial cell, epidermal cell, endothelial cell, vascular endothelial cell, hepatic parenchymal cell, chondrocyte, cumulus cell, nervous system cell, glial cell, neuron, oligodendrocyte, microglia, astroglia, heart cell, esophageal cell, muscle cell (for example, smooth muscle cell or skeletal muscle cell), pancreatic beta cell, melanocyte, hematopoietic progenitor cell, mononuclear cell, embryonic stem cell (ES cell), embryonal carcinoma cell, embryonic germ stem cell, induced pluripotent stem cell (iPS cell), nerve stem cell, hematopoietic stem cell, mesenchymal stem cell, hepatic stem cell, pancreatic stem cell, muscle stem cell, germline stem cell, intestinal stem cell, cancer stem cell, hair follicle stem cell, and various cell strains (for example, HCT116, Huh7, HEK293 (human fetal renal cell), HeLa (human cervical cancer cell strain), HepG2 (human liver cancer cell strain), UT7/TPO (human leukemia cell strain), CHO (Chinese hamster ovary cell strain), MDCK, MDBK, BHK, C-33A, HT-29, AE-1, 3D9, Ns0/1, Jurkat, NIH3T3, PC12, S2, Sf9, Sf21, High Five, and Vero).

Having an ability to inhibit adhesion of cells means that cell adhesion and spread are not observed by microscopic observation, and a cell aggregate (spheroid) is formed.

Alternatively, having the ability to inhibit adhesion of cells means that a luminescence intensity (%) (luminescence intensity of cells adhering to coating film of Example)/(luminescence intensity of cells adhering to well without coating) is 50% or less, preferably 30% or less, and more preferably 10% or less as compared with a case without coating with an ATP assay.

Having an ability to inhibit adsorption of a protein means that a relative average absorbance (%) ((average absorbance in Example)/(average absorbance without coating film)) is 80% or less, preferably 50% or less, and more preferably 20% or less as compared with a case without a coating film in IgG antibody HRP measurement performed by the method described in Examples.

The biological substance is preferably a cell and a protein.

### (Coating film)

The coating film of the present invention is obtained by applying the composition for forming a coating film of the present invention. In other words, the coating film of the present invention is a coating film of the composition for forming a coating film of the present invention.

The coating film is used to suppress adhesion of a biological substance.

Note that the coating film of the present invention is hardly dissolved in phosphate buffered saline, but the application of the coating film of the present invention is not particularly limited as long as it is used to suppress adhesion of a biological substance, and is not limited to applications in which it comes into contact with phosphate buffered saline.

In the present invention, the molar ratio of the repeating unit (A) to the repeating unit (B) in the copolymer is set to be within a specific range in order to obtain a coating film that is hardly dissolved in phosphate buffered saline. Therefore, in the present invention, a coating film that is hardly dissolved in phosphate buffered saline can be obtained without crosslinking of the copolymer. Therefore, the copolymer may not be crosslinked in the coating film. Since it is not required to perform light irradiation for crosslinking the copolymer when forming the coating film, a process of forming a coating film can be simplified.

A content of the copolymer in the coating film is not particularly limited, and is preferably 80 mass% or more, more preferably 90 mass% or more, and particularly preferably 95 mass% or more.

A thickness of the coating film is not particularly limited, and is, for example, 1 to 10,000 nm, preferably 5 to 1,000 nm, more preferably 10 to 500 nm, still more preferably 20 to 300 nm, further still more preferably 50 to 250 nm, and particularly preferably 100 to 250 nm.

Examples of a method for obtaining a coating film include a method for manufacturing a coating film of the present invention described below.

### (Substrate with coating film)

A substrate with a coating film of the present invention includes a substrate and the coating film of the present invention disposed on the substrate.

The coating film may be disposed on at least a part of the substrate, and does not need to be disposed on the entire surface of the substrate.

In a case where the coating film is used to suppress adhesion of cells in cell culture, the coating film is preferably formed on the entire surface on which the cell culture is performed.

The coating film is usually disposed on a surface of the substrate on which the adhesion suppression of the biological substance is required.

The substrate with a coating film can be obtained, for example, by a method for manufacturing a coating film of the present invention described below.

### (Method for manufacturing coating film)

A method for manufacturing a coating film of the present invention includes at least a supply step and a drying step, and further includes other steps such as a washing step and a sterilization step as necessary.

Note that the coating film of the present invention may be manufactured without performing the drying step after the supply step is performed.

### <Supply step>

The supply step is not particularly limited as long as it is a step of supplying the composition for forming a coating film onto a substrate.

### <<Substrate>>

A material, size, shape, and structure of the substrate are not particularly limited.

Examples of the material of the substrate include glass, a metal, a metal-containing compound or a metalloid-containing compound, activated carbon, and a resin.

Examples of the metal include typical metals: (alkali metals: Li, Na, K, Rb, and Cs; and alkaline earth metals Ca, Sr, Ba, and Ra), magnesium group elements: Be, Mg, Zn, Cd, and Hg; aluminum group elements: Al, Ga, and In; rare earth elements: Y, La, Ce, Pr, Nd, Sm, and Eu; tin group elements: Ti, Zr, Sn, Hf, Pb, and Th; iron group elements: Fe, Co, and Ni; earth-acid elements: V, Nb, and Ta, chromium group elements: Cr, Mo, W, and U; manganese elements: Mn and Re; noble metals: Cu, Ag, and Au; and platinum group elements: Ru, Rh, Pd, Os, Ir, and Pt.

Examples of the metal-containing compound or the metalloid-containing compound include ceramics which are a sintered body that contains a metal oxide as a basic component and is sintered by a heat treatment at a high temperature, semiconductors such as silicon, inorganic solid materials such as a shaped article of an inorganic compound, such as a metal oxide or metalloid oxide (such as silicon oxide or alumina), a metal carbide or metalloid carbide, a metal nitride or metalloid nitride (such as silicon nitride), or a metal boride or metalloid boride, aluminum, nickel titanium, and stainless steel (such as SUS304, SUS316, SUS316L, or the like).

The resin may be either a natural resin or a derivative thereof, or a synthetic resin, as the natural resin or the derivative thereof, cellulose, cellulose triacetate (CTA), nitrocellulose (NC), cellulose having dextran sulfate fixed thereto, or the like is preferably used, and as the synthetic resin, polyacrylonitrile (PAN), polyester polymer alloy (PEPA), polystyrene (PS), polysulfone (PSF), polyethylene terephthalate (PET), polymethyl methacrylate (PMMA), polyvinyl alcohol (PVA), polyurethane (PU), ethylene vinyl alcohol (EVAL), polyethylene (PE), polyester, polypropylene (PP), polyvinylidene fluoride (PVDF), polyether sulfone (PES), polycarbonate (PC), polyvinyl chloride (PVC), polytetrafluoroethylene (PTFE), ultra high molecular weight polyethylene (UHPE), polydimethylsiloxane (PDMS), an acrylonitrile-butadienestyrene resin (ABS), or Teflon (registered trademark) is preferably used. In the method for manufacturing a coating film of the present invention, when the composition for forming a coating film is coated so as to be present on at least a part of the surface of the substrate, a treatment at a high temperature is not required, and therefore, a resin or the like having low heat resistance can also be applied.

The material of the substrate may be one kind or a combination of two or more kinds.

Among these materials, glass, silicon, silicon oxide, polystyrene (PS), polypropylene (PP), polyethersulfone (PES), polyethylene terephthalate (PET), polycarbonate (PC), polyvinyl chloride (PVC), Teflon (registered trademark), cycloolefin polymer (COP), polydimethylsiloxane (PDMS), or stainless steel (SUS304, SUS316, SUS316L, or the like) alone, or a combination of two or more selected therefrom is preferable, and glass, polystyrene (PS), polypropylene (PP), polycarbonate (PC), stainless steel (SUS304, SUS316, SUS316L, or the like), and polydimethylsiloxane (PDMS) are particularly preferable.

The shape of the substrate is not particularly limited, and the substrate may be a flat plate having a flat surface, or may be a vessel-shaped substrate having a structure with a recess such as a well or a dish (so-called cell culture vessel).

Examples of the substrate include dishes (petri dishes) such as a petri dish, a tissue culture dish, and a multi-dish generally used for culturing cells, flasks such as a cell culture flask and a spinner flask, bags such as a plastic bag, Teflon (registered trademark) bag, and a culture bag, plates such as a microplate, a microwell plate, a multi-plate, and a multi-well plate, bottles such as a chamber slide, a tube, a tray, and a roller bottle, for example, a stirred cell culturing apparatus for culturing a large amount of cells, a cell culturing apparatus, and the like. Preferred examples thereof include a dish, a plate, and a tray.

### <<Supply method>>

A method for supplying the composition for forming a coating film onto a substrate is not limited, and examples thereof include a general coating method.

Examples of the coating method include a spin coating method, an inkjet method, a screen printing method, a slit coating method, a roll-to-roll method, a dip coating method, and a solvent casting method. The coating method is preferably a solvent casting method, an inkjet method, or a printing technique such as screen printing.

More specific examples of the coating method include a method of immersing a substrate in a composition for forming a coating film, and a method of adding a composition for forming a coating film to a substrate and allowing the substrate to stand for a predetermined time.

In a case where the substrate is a vessel, the supply method can be performed by, for example, a method of adding a composition for forming a coating film to a vessel and allowing the vessel to stand for a predetermined time, or a method of adding a composition for forming a coating film to a substrate, allowing the substrate to stand for a predetermined time, and drying the substrate after drainage. The addition can be performed, for example, by adding the composition for forming a coating film in an amount of 0.5 to 1 times the total volume of the vessel using a syringe or the like.

### <Drying step>

The drying step is not particularly limited as long as it is a step of drying the composition for forming a coating film supplied onto the substrate.

The drying can be performed, for example, in a range of a temperature of -200°C to 200°C under the atmosphere or under vacuum.

The coating film can be formed, for example, by drying at room temperature (10°C to 35°C, for example, 25°C), and may be dried, for example, at 40°C to 100°C in order to form the coating film more quickly.

A preferred drying temperature is 10°C to 180°C, and a more preferred drying temperature is 20°C to 150°C.

A drying time is not particularly limited, and is, for example, 1 minute to 24 hours.

### <Washing step>

The washing step is not particularly limited as long as it is a step of washing the coating film obtained by the drying step. The washing step is performed to remove, for example, impurities, unreacted monomers, and the like remaining in the coating film from the coating film.

The washing can be performed by a known method, and flowing water washing and ultrasonic washing are preferable.

Examples of a solvent used for washing include water and an aqueous solution containing an electrolyte. The solvent is usually used at room temperature (for example, 10 to 35°C), and may be heated to, for example, a range of 40°C to 95°C. The aqueous solution containing an electrolyte is preferably PBS, physiological saline (containing only sodium chloride), Dulbecco's phosphate buffered saline, Tris-buffered saline, HEPES-buffered saline, or Veronal-buffered saline, and particularly preferably PBS.

The coating film of the present invention remains firmly fixed to the substrate without being eluted even when washed with water, PBS, an alcohol, or the like.

In addition, in a case where the coating film is used for cell culture, washing may be performed using a medium (for example, water, a buffer solution, a medium, or the like) of a sample used for cell culture. A preferred medium is a medium, and a more preferred medium is BME medium (Eagle's basal medium) or DMEM medium (Dulbecco's modified eagle medium).

### <Sterilization step>

In a case where the coating film is used in a cell culture apparatus, the coating film needs to undergo a sterilization step by irradiation with radiation.

The sterilization step is usually performed at ambient temperature (for example, about 0°C to about 40°C, preferably about 10°C to about 30°C, and preferably about 25°C). The radiation to be irradiated is not limited as long as sterilization can be performed, and γ-ray, X-ray, or electron beam irradiation is preferable. A γ-ray or an X-ray is more preferable, and a γ-ray is still more preferable. An irradiation dose of the γ-ray may be, for example, a dose employed in a normal sterilization step, and for example, irradiation of about 5 to 40 kGy is sufficient, and irradiation of 10 to 25 kGy is preferable.

### (Cell culture vessel)

A cell culture vessel of the present invention includes the substrate with a coating film of the present invention.

The substrate with a coating film itself may be a cell culture vessel.

The cell culture vessel can be used, for example, for producing a cell aggregate.

The cell culture vessel may have a cell culture base film having cell adhesiveness, the cell culture base film being formed on the coating film.

Examples of the cell culture base film include the cell culture base film described in WO 2020/040247 A. Note that the contents of WO 2020/040247 A are hereby incorporated in their entireties by reference, to the extent that they have been disclosed herein.

An example of the base film described in WO 2020/040247 A is a base film containing a polymer containing at least a repeating unit derived from a monomer represented by the following Formula (I). The polymer may contain a repeating unit derived from a monomer represented by the following Formula (II). 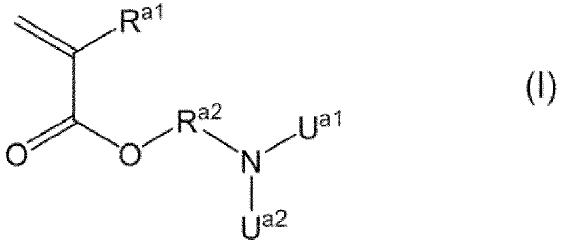 (In Formula (I), U^{a1} and U^{a2} each independently represent a hydrogen atom or an alkyl group having 1 to 5 carbon atoms, R^{a1} represents a hydrogen atom or an alkyl group having 1 to 5 carbon atoms, and R^{a2} represents an alkylene group having 1 to 5 carbon atoms.) 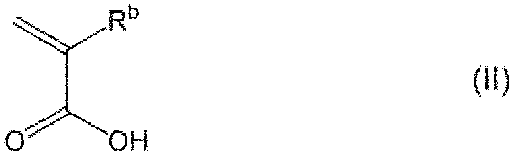 (In Formula (II), R^{b} represents a hydrogen atom or an alkyl group having 1 to 5 carbon atoms.)

Specific examples of the alkylene group having 1 to 5 carbon atoms include the specific examples of the alkylene group having 1 to 5 carbon atoms exemplified in the descriptions of Formula (A) and Formula (B).

The cell culture base film is not uniformly disposed on the coating film, but is disposed in a pattern, for example.

Examples of the pattern include a dot pattern.

In other words, an example of the cell culture vessel has a cell seeding surface having a coating film that can suppress cell adhesion and a cell culture base film having cell adhesiveness disposed in a dot pattern on the coating film.

A thickness of the cell culture base film is, for example, 1 to 1,000 nm, and is preferably 5 to 500 nm, preferably 5 to 300 nm, preferably 5 to 200 nm, preferably 5 to 150 nm, preferably 10 to 150 nm, or preferably 20 to 150 nm.

### (Method for producing cell aggregate)

A method for producing a cell aggregate includes a step of seeding cells in the cell culture vessel of the present invention, and further includes other steps as necessary.

For example, the cells are seeded on the coating film of a well-shaped cell culture vessel, such that adhesion, spread, and proliferation of the cells are suppressed by the cell adhesion suppression function of the coating film, and as a result, an excellent three-dimensional cell aggregate (for example, spheroid) is obtained.

For example, the cell culture vessel has a cell seeding surface having a coating film that can suppress cell adhesion and a cell culture base film having cell adhesiveness disposed in a dot pattern on the coating film.

The cells are seeded on such a cell seeding surface, such that the cells are selectively cultured on the cell culture base film with a dot pattern, and as a result, a three-dimensional cell aggregate (for example, spheroid) is obtained.

### Examples

Hereinafter, the present invention will be described in more detail with reference to Examples and the like, but the present invention is not limited by the following Examples and the like.

### <Example 1>

### (Preparation of composition for forming coating film)

Polyvinyl acetate (manufactured by JAPAN VAM & POVAL CO., LTD., JMR-10L (registered trademark) (polymerization degree: 250, saponification degree: 35.8%)) was dissolved in water/ethanol (3/7 mass ratio) so as to have a concentration of 10 mg/g, thereby preparing a composition for forming a coating film. The obtained composition for forming a coating film was transparent and uniform.

### (Formation of coating film on HMDS-treated silicon wafer)

The obtained composition for forming a coating film was spin-coated on a 1,1,1,3,3,3-hexamethyldisilazane (HMDS)-treated silicon wafer at 1,500 rpm/60 sec, and drying was performed in an oven at 70°C for 24 hours as a drying step, thereby obtaining a coating film formed on the HMDS-treated silicon wafer. A film thickness of the coating film formed on the HMDS-treated silicon wafer was measured using a spectroscopic ellipsometer. Thereafter, washing was sufficiently performed with phosphate buffered saline (PBS), drying was performed in an oven at 50°C for 1 hour, and a film thickness of the coating film formed on the HMDS-treated silicon wafer was measured using a spectroscopic ellipsometer. A residual film rate was calculated from the film thickness after PBS washing with respect to the film thickness after coating. The results were shown in Table 1-1.

### (Production of coating plate for cell culture)

A coating plate for cell culture was produced by the following method (i), (ii), or (iii) using the composition for forming a coating film.

### (i) (Examples 1 and 2 and Comparative Examples 1, 2, 5, and 6)

The composition for forming a coating film was added to separate wells of a 96-well cell culture plate (manufactured by Corning Inc., #351172, volume: 0.37 mL, formed of polystyrene) at 200 µL/well, the plate was allowed to stand at room temperature for 1 hour, and then an excess composition was removed.

Thereafter, drying was performed at 70°C for 24 hours using an oven.

Thereafter, each coated well was washed 3 times with 250 µL of pure water, drying was performed for 1 hour using an oven at 50°C, and then the dried well was used for the test.

### (ii) (Examples 8, 9, and 20 and Comparative Examples 3 and 4)

The composition for forming a coating film was added to separate wells of a 24-well cell culture plate (manufactured by Corning Inc., #351147, volume: 1 mL, formed of polystyrene) at 800 µL/well, the plate was allowed to stand at room temperature for 1 hour, and then an excess composition was removed.

Thereafter, drying was performed at 70°C for 24 hours using an oven.

Thereafter, each coated well was washed 3 times with 1.5 mL of pure water, drying was performed for 1 hour using an oven at 50°C, and then the dried well was used for the test.

### (iii) (Examples 3 to 7, 10 to 19, and 21 to 27)

The composition for forming a coating film was added to separate wells of a 24-well cell culture plate (manufactured by Corning Inc., #351147, volume: 1 mL, formed of polystyrene) at 100 µL/well, the plate was allowed to stand at room temperature for 3 hours, and then drying was performed at 70°C for 24 hours using an oven.

### (Preparation of cells)

A cell suspension was prepared by the following method (iv) or (v).

### (iv) (Examples 1 to 27 and Comparative Examples 1 to 6)

As cells, mouse embryonic fibroblasts (manufactured by DS Pharma Biomedical Co., Ltd.) were used. As a medium used for cell culture, BME medium (manufactured by Thermo Fisher Scientific Inc.) containing 10% FBS (manufactured by Sigma-Aldrich) and an L-glutamine-penicillin-streptomycin stabilization solution (manufactured by Thermo Fisher Scientific Inc.) was used. The cells were statically cultured for 2 days or longer using a petri dish (medium 10 mL) having a diameter of 10 cm in a 37°C/CO₂ incubator in a state where a concentration of carbon dioxide was maintained at 5%. Subsequently, the present cells were washed with 5 mL of PBS, 1 mL of a 0.25 w/v % trypsin-1 mmol/L EDTA solution (manufactured by FUJIFILM Wako Pure Chemical Corporation) was added to detach the cells, and then the cells were suspended in 10 mL of the medium. The present suspension was centrifuged (manufactured by Tomy Seiko Co., Ltd., model number LC-200, 1,000 rpm/3 min, room temperature), the supernatant was removed, and then the medium was added, thereby preparing a cell suspension.

The prepared cell suspension was used for cell adhesion tests of Examples 1 to 27 and Comparative Examples 1 to 6.

In addition, the prepared cell suspension was used for cell aggregate formation tests of Examples 3, 4, 7, 13 to 15, 18, 23, and 24.

### (v) (Examples 10 to 12, 19 to 22, and 25 to 30)

As cells, human adipose tissue-derived mesenchymal stem cells ADSC (manufactured by CellSource Co., Ltd.) were used. As a medium used for culturing cells, Mesenchymal Stem Cell Growth Medium 2 (manufactured by PromoCell GmbH) as a low serum medium containing an L-glutamine-penicillin-streptomycin stabilization solution (manufactured by Thermo Fisher Scientific Inc.) was used. The cells were statically cultured for 2 days or longer using a petri dish (medium 10 mL) having a diameter of 10 cm in a 37°C/CO₂ incubator in a state where a concentration of carbon dioxide was maintained at 5%. Subsequently, the present cells were washed with 5 mL of PBS, 1 mL of TrypLE Select Enzyme (manufactured by Thermo Fisher Scientific Inc.) was added to detach the cells, and each of the cells was suspended in 10 mL of the medium. The present suspension was centrifuged (manufactured by Tomy Seiko Co., Ltd., model number LC-200, 1,000 rpm/3 min, room temperature), the supernatant was removed, and then the medium was added, thereby preparing a cell suspension.

The prepared cell suspension was used for cell adhesion tests of Examples 28 to 30.

The prepared cell suspension was used for cell aggregate formation tests of Examples 10 to 12, 19 to 22, and 25 to 30.

### (Cell adhesion test)

To each well of the plate produced above, 150 µL of each cell suspension was added so as to be 1 × 10⁴ cells/well in a 96-well cell culture plate, and 0.5 mL of each cell suspension was added so as to be 5 × 10⁴ cells/well in a 24-well cell culture plate. Thereafter, the suspension was allowed to stand in a CO₂ incubator at 37°C for 3 days in a state where a concentration of carbon dioxide was maintained at 5%. After 3 days of culture, adhesion of the cells to each well of the plate produced above was compared based on observation with an inverted microscope (manufactured by Olympus Corporation, CKX31) (magnification: 4 times).

The observation results were evaluated according to the following evaluation criteria, and the cell adhesion suppression effect was confirmed. The results were shown in Table 1-1.

### [Evaluation criteria]

o: There is no adhesion or spread of the cells to the bottom surface of the substrate, and a state in which cell aggregates (spheroids) are formed in the well is observed.
×: A state in which the cells adhere and spread to the bottom surface of the substrate is observed.

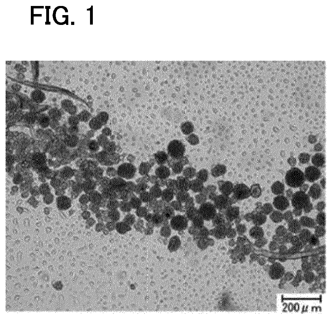

A photograph of a microscopic observation result of a cell adhesion test in Example 1 was illustrated in Fig. 1.

In the coating film obtained using the composition for forming a coating film of Example 1, adhesion and spread of the cells were not observed by hydrophilizing the substrate surface, and formation of cell aggregates (spheroids) was observed in the well.

### (Production of coating plate for protein adsorption test)

The composition for forming a coating film was added to 5 wells of separate wells of a 96-well cell culture plate (manufactured by Corning Inc., #9017, volume: 0.36 mL, formed of polystyrene) at 150 µL/well, drainage was performed after immersion at room temperature for 1 hour, and then drying was performed at 50°C for 24 hours using an oven. Thereafter, each coated well was washed 3 times with 200 µL of pure water, drying was performed for 1 hour using an oven at 70°C, and then the dried well was used for the test.

As a negative control, wells of an uncoated 96-well cell culture plate (manufactured by Corning Inc., #9017, volume 0.36 mL, formed of polystyrene) were used.

### (Preparation of IgG-HRP diluted solution)

A goat anti-mouse IgG antibody HRP conjugate (manufactured by Southern Biotechnology Associates) was diluted with PBS so as to have a concentration of 1 mg/g, thereby preparing an IgG-HRP diluted solution.

### (Protein adsorption test)

To each well of the plate produced above, for the negative control, the IgG-HRP diluted solution was added at 100 µL/well, and the plate was allowed to stand at room temperature for 30 minutes. After 30 minutes, the IgG-HRP diluted solution was drained, and each well was washed 3 times with 200 µL of PBS. TMB solution (manufactured by SeraCare Life Sciences Inc., SureBlue) was added at 100 µL/well, and after 1 minute, TMB STOP solution (manufactured by SeraCare Life Sciences Inc.) was added at 100 µL/well. An absorbance was measured at 450 nm and 650 nm using a microplate reader (manufactured by Tecan Group Ltd., infinite M200PRO). A value obtained by subtracting the absorbance at 650 nm from the absorbance at 450 nm was calculated to obtain an average absorbance of 5 wells. The average absorbance in the wells of the negative control was defined as a protein adsorption rate of 100%, and the protein adsorption rate of the wells coated with the composition for forming a coating film was calculated. The results were shown in Table 2.

### <Example 2 and Comparative Examples 1 and 2>

A composition for forming a coating film was prepared in the same manner as that of Example 1, except that the polymerization degree and the saponification degree of polyvinyl acetate and the composition of each solvent were changed as described in Table 1-1 or Table 1-2. The obtained composition for forming a coating film was transparent and uniform.

The obtained composition for forming a coating film was operated in the same manner as that of Example 1 to form a coating film on an HMDS-treated silicon wafer and to produce a coating plate for cell culture and a coating plate for a protein adsorption test.

A residual film rate was determined in the same manner as that of Example 1.

A cell adhesion test was performed in the same manner as that of Example 1.

A protein adsorption test was performed in the same manner as that of Example 1.

The results were shown in Table 1-1 or Table 1-2 and Table 2.

### <Example 3>

A composition for forming a coating film was prepared in the same manner as that of Example 1, except that the polymerization degree and the saponification degree of polyvinyl acetate and the composition of each solvent were changed as described in Table 1-1. The obtained composition for forming a coating film was transparent and uniform.

The obtained composition for forming a coating film was operated in the same manner as that of Example 1 to form a coating film on an HMDS-treated silicon wafer.

In addition, a coating plate for cell culture was produced by the method (iii).

In addition, a coating plate for a protein adsorption test was produced by the same operation as that of Example 1.

A residual film rate was determined in the same manner as that of Example 1.

A cell adhesion test was performed in the same manner as that of Example 1.

A protein adsorption test was performed in the same manner as that of Example 1.

The results were shown in Table 1-1 and Table 2.

Next, a cell culture vessel including a cell culture base film formed on the coating film for cell culture was produced by the following operation.

### (Synthesis of polymer for forming cell culture base film)

24.00 g of 2-(dimethylamino)ethyl methacrylate (manufactured by Tokyo Chemical Industry Co., Ltd.), 1.46 g of methacrylic acid (manufactured by Tokyo Chemical Industry Co., Ltd.), 5.09 g of ethylene glycol dimethacrylate (manufactured by Tokyo Chemical Industry Co., Ltd.), 0.31 g of dimethyl-1,1'-azobis(1-cyclohexanecarboxylate) (VE-073, manufactured by FUJIFILM Wako Pure Chemical Corporation), and 111.09 g of 2-propanol were mixed, and dropwise polymerization was performed with 166.62 g of 2-propanol at a reflux temperature, thereby synthesizing a polymer. A reaction product was reprecipitated with hexane as a poor solvent, and the precipitate was collected by filtration and dried under reduced pressure.

A weight-average molecular weight of the polymer by GFC was 228,000.

### (Preparation of cell culture base film forming agent)

To 0.0466 g of the polymer obtained above, 46.6 g of pure water was added and sufficiently stirred to prepare a dilution composition. To 0.7006 g of the dilution composition obtained above, 3.6916 g of sterile water and 0.2796 g of Recombinant Human Vitronectin (manufactured by PeproTech, Inc.) diluted to 0.5 mg/mL with sterile water were added and sufficiently stirred, thereby preparing a cell culture base film forming agent.

### (Production of plate for cell aggregate formation by inkjet)

Using an inkjet apparatus (manufactured by Microjet Technology Co., Ltd., model number: LaboJet-600) and an inkjet head (model number: IJHBS-1000), an appropriate amount of the cell culture base film forming agent prepared above was applied to the coating plate for cell culture produced by the method (iii). Drying was performed in an oven at 70°C for 24 hours to produce a plate for cell aggregate formation.

### (Preparation of cells)

A cell suspension was prepared by the method (iv) in the same manner as that of Example 1.

### (Cell aggregate formation test)

To each well of the plate for cell aggregate formation produced above, 350 µL of the cell suspension was added so as to be 25 × 10⁴ cells/cm². Thereafter, the suspension was allowed to stand for 2 hours in a CO₂ incubator at 37°C for 3 days in a state where a concentration of carbon dioxide was maintained at 5%. After standing, the appearance of the adherent cells was observed and imaged using an inverted microscope (manufactured by Olympus Corporation, CKX31). Furthermore, on the next day, the presence or absence of cell aggregate on the cell culture base film was observed and imaged.

The observation results were evaluated according to the following evaluation criteria. The results were shown in Table 3.

### [2 Hours after seeding, cell adhesion]

### [[Evaluation criteria]]

o: 2 Hours after seeding, a state in which the cells selectively adhere and spread only to the cell culture base film is observed.
×: 2 Hours after seeding, a state in which the cells non-selectively adhere and spread to the bottom surface of the cell culture substrate is observed.

### [One day after seeding, cell aggregate formation]

### [[Evaluation criteria]]

o: 2 Hours after seeding, a state in which the cells selectively adhere and spread to only the cell culture base film is observed, and one day after seeding, a state in which the cells are aggregated on the cell culture base film and cell aggregates are formed is observed.
×: 2 Hours after seeding, a state in which the cells non-selectively adhere and spread to the bottom surface of the cell culture substrate is observed, and one day after seeding, a state in which the cells non-selectively adhere to the bottom surface of the cell culture substrate and the spread state is maintained is observed.

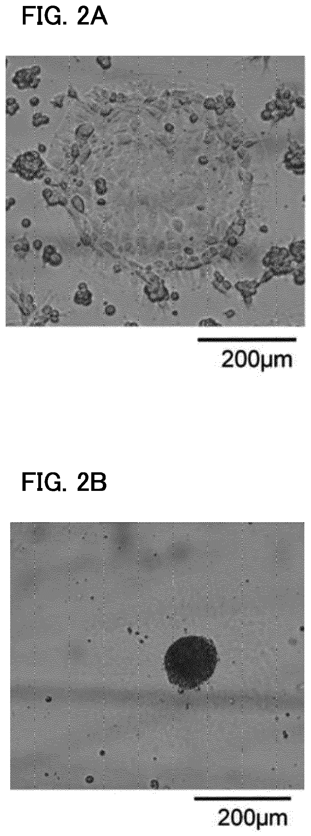

A photograph of a microscopic observation result of cell aggregates after cell adhesion 2 hours after seeding in Example 3 was illustrated in Fig. 2A.

A photograph of a microscopic observation result of cell aggregates one day after seeding in Example 3 was illustrated in Fig. 2B.

In the plate for cell aggregate formation obtained by forming the cell culture base film on the coating film obtained using the composition for forming a coating film of Example 3, it was confirmed that the cells selectively adhered to the cell culture base film 2 hours after seeding, and after one day, the cells were detached, and cell adhesion aggregates were formed.

### <Examples 4, 7, and 13>

A composition for forming a coating film was prepared in the same manner as that of Example 1, except that the polymerization degree and the saponification degree of polyvinyl acetate and the composition of each solvent were changed as described in Table 1-1. The obtained composition for forming a coating film was transparent and uniform.

The obtained composition for forming a coating film was operated in the same manner as that of Example 3 to form a coating film on an HMDS-treated silicon wafer and to produce a coating plate for cell culture, a plate for cell aggregate formation, and a coating plate for a protein adsorption test.

A residual film rate was determined in the same manner as that of Example 1.

A cell adhesion test was performed in the same manner as that of Example 1.

A cell aggregate formation test was performed in the same manner as that of Example 3.

A protein adsorption test was performed in the same manner as that of Example 1.

The results were shown in Table 1-1, Table 2, and Table 3.

### <Examples 5 and 6>

A composition for forming a coating film was prepared in the same manner as that of Example 1, except that the polymerization degree and the saponification degree of polyvinyl acetate and the composition of each solvent were changed as described in Table 1-1. The obtained composition for forming a coating film was transparent and uniform.

The obtained composition for forming a coating film was operated in the same manner as that of Example 3 to form a coating film on an HMDS-treated silicon wafer and to produce a coating plate for cell culture and a coating plate for a protein adsorption test.

A residual film rate was determined in the same manner as that of Example 1.

A cell adhesion test was performed in the same manner as that of Example 1.

A protein adsorption test was performed in the same manner as that of Example 1.

The results were shown in Table 1-1 and Table 2.

### <Examples 8 and 9 and Comparative Examples 3 and 4>

A composition for forming a coating film was prepared in the same manner as that of Example 1, except that the polymerization degree and the saponification degree of polyvinyl acetate and the composition of each solvent were changed as described in Table 1-1 or Table 1-2. The obtained composition for forming a coating film was transparent and uniform.

The obtained composition for forming a coating film was operated in the same manner as that of Example 1 to form a coating film on an HMDS-treated silicon wafer.

In addition, a coating plate for cell culture was produced by the method (ii).

In addition, a coating plate for a protein adsorption test was produced by the same operation as that of Example 1.

A residual film rate was determined in the same manner as that of Example 1.

A cell adhesion test was performed in the same manner as that of Example 1.

A protein adsorption test was performed in the same manner as that of Example 1.

The results were shown in Table 1-1 or Table 1-2 and Table 2.

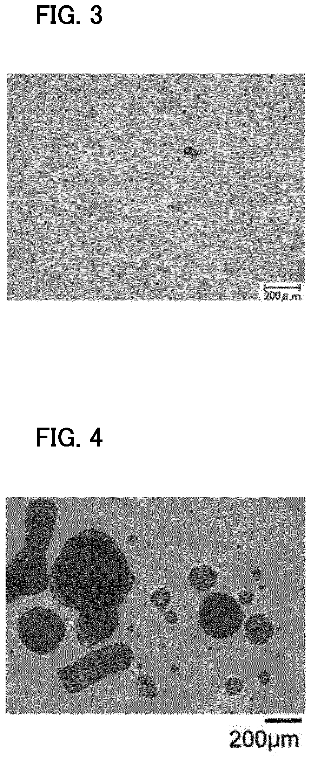

Fig. 3 is a photograph of a microscopic observation result of a cell adhesion test in Comparative Example 3.

In the coating film obtained using the composition for forming a coating film of Comparative Example 3, the surface of the substrate was not sufficiently hydrophilized, and the cells adhered and spread to the bottom surface of the plate.

### <Examples 10, 11, and 12>

A composition for forming a coating film was prepared in the same manner as that of Example 1, except that the polymerization degree and the saponification degree of polyvinyl acetate and the composition of each solvent were changed as described in Table 1-1. The obtained composition for forming a coating film was transparent and uniform.

The obtained composition for forming a coating film was operated in the same manner as that of Example 3 to form a coating film on an HMDS-treated silicon wafer and to produce a coating plate for cell culture, a plate for cell aggregate formation, and a coating plate for a protein adsorption test.

A residual film rate was determined in the same manner as that of Example 1.

A cell adhesion test was performed in the same manner as that of Example 1.

A cell aggregate formation test was performed in the same manner as that of Example 3, except that the cell suspension was prepared by the method (v).

A protein adsorption test was performed in the same manner as that of Example 1.

The results were shown in Table 1-1, Table 2, and Table 3.

### <Examples 14, 15, 18, 23, and 24>

A composition for forming a coating film was prepared in the same manner as that of Example 1, except that the polymerization degree and the saponification degree of polyvinyl acetate and the composition of each solvent were changed as described in Table 1-1 or Table 1-2. The obtained composition for forming a coating film was transparent and uniform.

The obtained composition for forming a coating film was operated in the same manner as that of Example 3 to form a coating film on an HMDS-treated silicon wafer and to produce a coating plate for cell culture, a plate for cell aggregate formation, and a coating plate for a protein adsorption test. Thereafter, a γ-ray (25 kGy) was irradiated.

A residual film rate was determined in the same manner as that of Example 1.

A cell adhesion test was performed in the same manner as that of Example 1.

A cell aggregate formation test was performed in the same manner as that of Example 3.

A protein adsorption test was performed in the same manner as that of Example 1.

The results were shown in Table 1-1 or Table 1-2 and Table 2 and Table 3.

### <Examples 16 and 17>

A composition for forming a coating film was prepared in the same manner as that of Example 1, except that the polymerization degree and the saponification degree of polyvinyl acetate and the composition of each solvent were changed as described in Table 1-1. The obtained composition for forming a coating film was transparent and uniform.

The obtained composition for forming a coating film was operated in the same manner as that of Example 14 to form a coating film on an HMDS-treated silicon wafer and to produce a coating plate for cell culture and a coating plate for a protein adsorption test, and then a γ-ray (25 kGy) was irradiated.

A residual film rate was determined in the same manner as that of Example 1.

A cell adhesion test was performed in the same manner as that of Example 1.

A protein adsorption test was performed in the same manner as that of Example 1.

The results were shown in Table 1-1 and Table 2.

### <Examples 19, 21, 22, and 25 to 27>

A composition for forming a coating film was prepared in the same manner as that of Example 1, except that the polymerization degree and the saponification degree of polyvinyl acetate and the composition of each solvent were changed as described in Table 1-2. The obtained composition for forming a coating film was transparent and uniform.

The obtained composition for forming a coating film was operated in the same manner as that of Example 14 to form a coating film on an HMDS-treated silicon wafer and to produce a coating plate for cell culture, a plate for cell aggregate formation, and a coating plate for a protein adsorption test, and then a γ-ray (25 kGy) was irradiated.

A residual film rate was determined in the same manner as that of Example 1.

A cell adhesion test was performed in the same manner as that of Example 1.

A cell aggregate formation test was performed in the same manner as that of Example 10.

A protein adsorption test was performed in the same manner as that of Example 1.

The results were shown in Table 1-2, Table 2, and Table 3.

### <Example 20>

A composition for forming a coating film was prepared in the same manner as that of Example 1, except that the polymerization degree and the saponification degree of polyvinyl acetate and the composition of each solvent were changed as described in Table 1-2. The obtained composition for forming a coating film was transparent and uniform.

The obtained composition for forming a coating film was operated in the same manner as that of Example 8 to form a coating film on an HMDS-treated silicon wafer and to produce a coating plate for cell culture, a plate for cell aggregate formation, and a coating plate for a protein adsorption test. Thereafter, a γ-ray (25 kGy) was irradiated.

A residual film rate was determined in the same manner as that of Example 1.

A cell adhesion test was performed in the same manner as that of Example 1.

A cell aggregate formation test was performed in the same manner as that of Example 10.

A protein adsorption test was performed in the same manner as that of Example 1.

The results were shown in Table 1-2, Table 2, and Table 3.

### <Example 28>

### (Preparation of composition for forming coating film)

A composition for forming a coating film was prepared in the same manner as that of Example 11, except that dissolution was performed so as to have a concentration of 3 mg/g. The obtained composition for forming a coating film was transparent and uniform.

### (Production of coating plate for cell culture by inkjet)

Using an inkjet apparatus (manufactured by Seiko Epson Corporation, R&D inkjet apparatus) and an inkjet head (manufactured by Seiko Epson Corporation, Precision Core Head S800-A1), an appropriate amount of the composition for forming a coating film prepared above was applied to a polystyrene substrate having a size of 79 mm × 121 mm in a perfect circle having a diameter of 18 mm. Drying was performed in an oven at 70°C for 24 hours. The resultant was attached to a bottomless 24-well plate (manufactured by CSTEC CO., LTD.) to produce a coating plate for cell culture.

### (Preparation of cells)

A cell suspension was prepared by the method (v) in the same manner as that of Example 10.

### (Cell adhesion test)

A cell adhesion test was performed in the same manner as that of Example 1, except that the cell suspension prepared by the method (v) was used.

### (Preparation of cell culture base film forming agent)

A cell culture base film forming agent was prepared in the same manner as that of Example 3.

### (Production of plate for cell aggregate formation by inkjet)

A plate for cell aggregate formation was produced in the same manner as that of Example 3, except that the coating plate for cell culture produced by the above method was used.

### (Cell aggregate formation test)

A cell aggregate formation test was performed in the same manner as that of Example 10, except that 500 µL of the cell suspension was added so as to be 25 × 10⁴ cells/cm².

The results were shown in Table 4.

A photograph of a microscopic observation result of the cell adhesion test was illustrated in Fig. 4.

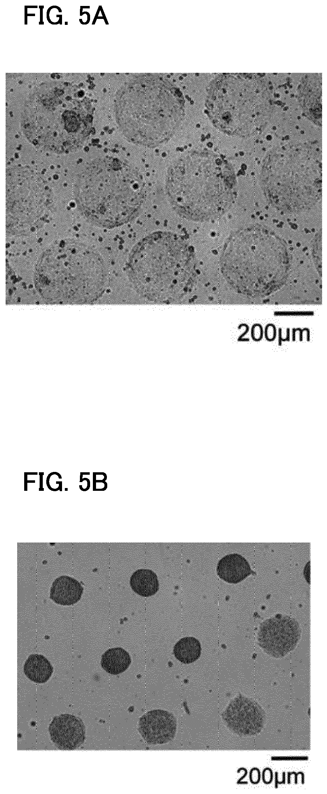

A photograph of a microscopic observation result of cell adhesion 2 hours after seeding in the cell aggregate formation test was illustrated in Fig. 5A.

A photograph of a microscopic observation result of cell aggregates one day after seeding in the cell aggregate formation test was illustrated in Fig. 5B.

In the coating film for cell culture obtained using the inkjet apparatus, adhesion and spread of the cells were not observed by hydrophilizing the substrate surface, and formation of cell aggregates (spheroids) was observed in the well.

In the plate for cell aggregate formation obtained by forming the cell culture base film on the coating film obtained using the inkjet apparatus, it was confirmed that the cells selectively adhered to the cell culture base film 2 hours after seeding, and after one day, the cells were detached, and cell adhesion aggregates were formed.

### <Example 29>

A composition for forming a coating film was prepared in the same manner as that of Example 12, except that dissolution was performed so as to have a concentration of 3 mg/g. The obtained composition for forming a coating film was transparent and uniform.

The obtained composition for forming a coating film was operated in the same manner as that of Example 28 to produce a coating plate for cell culture and a plate for cell aggregate formation.

A cell adhesion test was performed in the same manner as that of Example 28.

A cell aggregate formation test was performed in the same manner as that of Example 28.

The results were shown in Table 4.

### <Example 30>

A composition for forming a coating film was prepared in the same manner as that of Example 22, except that dissolution was performed so as to have a concentration of 3 mg/g. The obtained composition for forming a coating film was transparent and uniform.

The obtained composition for forming a coating film was operated in the same manner as that of Example 28 to produce a coating plate for cell culture and a plate for cell aggregate formation.

A cell adhesion test was performed in the same manner as that of Example 28.

A cell aggregate formation test was performed in the same manner as that of Example 28.

The results were shown in Table 4.

### <Examples 31 to 38>

A coating plate for cell culture was produced by any one of the following methods (vi) to (ix) using the composition for forming a coating film prepared in Example 10 or Example 11.

### (vi) (Examples 31 and 32)

The composition for forming a coating film was added to separate wells of a 96-well round-bottom cell culture plate (manufactured by Thermo Fisher Scientific Inc., #262162, volume 0.3 mL, formed of polystyrene) at 200 µL/well, the plate was allowed to stand at room temperature for 1 hour, and then an excess composition was removed.

Thereafter, drying was performed at 70°C for 24 hours using an oven.

Thereafter, each coated well was washed 3 times with 200 µL of pure water, drying was performed for 4 hours using an oven at 50°C, and then the dried well was used for the test.

### (vii) (Examples 33 and 34)

The composition for forming a coating film was added to separate wells of a 96-well round-bottom cell culture plate (manufactured by Thermo Fisher Scientific Inc., #262162, volume 0.3 mL, formed of polystyrene) at 16 µL/well, the plate was allowed to stand at room temperature for 1 hour, and then drying was performed at 70°C for 24 hours using an oven.

Thereafter, each coated well was washed 3 times with 200 µL of pure water, drying was performed for 4 hours using an oven at 50°C, and then the dried well was used for the test.

### (viii) (Examples 35 and 36)

A test plate obtained by the same operation as that of (vi) was irradiated with a γ-ray (15/30kGy) and used for a test.

### (ix) (Examples 37 and 38)

A test plate obtained by the same operation as that of (vii) was irradiated with a γ-ray (15/30kGy) and used for a test.

### (Preparation of human induced pluripotent stem cells)

For culturing human induced pluripotent stem cells (hiPSCs) 1383D2 strain (obtained from Kyoto University iPS Cell Research Institute), mTeSR (trademark) 1 (manufactured by STEMCELL Technologies Inc.) or StemFit (registered trademark) AK02N (manufactured by Ajinomoto Co., Inc.) was used. The cells were statically cultured for 2 days or longer in a 37°C/CO₂ incubator in a state in which a concentration of carbon dioxide was maintained at 5%, using a 6-well plate (medium: 2 mL) coated with Vitronectin (VTN-N) Recombinant Human Protein, Truncated (manufactured by Thermo Fisher Scientific Inc., #A14700) or iMatrix-511 silk (manufactured by Nippi. Inc., #387-10131) at 0.5 µg/cm². Subsequently, the present cells were washed with 2 mL of a PBS solution, 0.5 mL of TrypLE (trademark) Select Enzyme (1X) and no phenol red (manufactured by Thermo Fisher Scientific Inc.) were added to detach the cells as single cells, and each of the cells was suspended in 2 mL of the medium. The present suspension was centrifuged (manufactured by Tomy Seiko Co., Ltd., model number EIX-136, 200 × g./3 min, room temperature), the supernatant was removed, and then the medium was added, thereby preparing a cell suspension.

### (Preparation of adipose-derived stem cells)

For culturing adipose-derived stem cells (manufactured by CellSource Co., Ltd., hereinafter, referred to as ADSC), mesenchymal stem cell growth medium 2 (manufactured by PromoCell GmbH, #C-28009) was used as a low serum medium, or mesenchymal stem cell growth medium XF (manufactured by PromoCell GmbH, #C-28019) was used as a serum-free medium. The cells were statically cultured for 2 days or longer using a petri dish (medium 10 mL) having a diameter of 10 cm in a 37°C/CO₂ incubator in a state where a concentration of carbon dioxide was maintained at 5%. Subsequently, the present cells were washed with 5 mL of a PBS solution, 1 mL of TrypLE (trademark) Select (1X) (manufactured by Thermo Fisher Scientific Inc., #12563011) was added to detach the cells, and each of the cells was suspended in 10 mL of the medium. The present suspension was centrifuged (manufactured by Tomy Seiko Co., Ltd., model number EIX-136, 220 × g./3 min, room temperature), the supernatant was removed, and then the medium was added, thereby preparing a cell suspension.

### (Spheroid formation experiment)

To each well of the plate produced above, 100 µL of each cell suspension was added so as to be 2,000 cells/well in a 96-well cell culture plate. Thereafter, the suspension was allowed to stand in a CO₂ incubator at 37°C overnight in a state where a concentration of carbon dioxide was maintained at 5%. On the next day, the presence or absence of spheroid formation in each well of the plate produced above was compared based on observation with an inverted microscope (manufactured by Olympus Corporation, #IX-73) (magnification: 4 times).

The observation results were evaluated according to the following evaluation criteria, and the spheroid formation effect was confirmed.

### [Evaluation criteria]

ο: A state in which the seeded cells are aggregated into one at the bottom of the well is observed.
×: A state in which the seeded cells are not aggregated into one at the bottom of the well, but form a plurality of small clumps is observed.

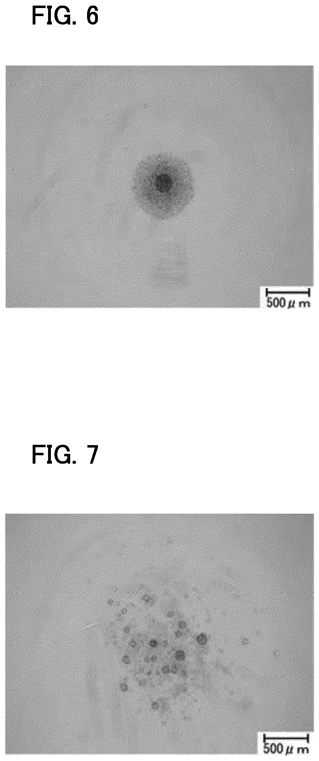

A photograph of a microscopic observation result of the spheroid formation experiment of the human induced pluripotent stem cells using mTeSR (trademark) 1 of Example 31 was illustrated in Fig. 6.

The results of the spheroid formation experiment are shown in Table 5. In the coating films obtained using the compositions for forming a coating film of Examples 31 to 38, adhesion and spread of the cells were not observed, and formation of cell aggregates (spheroids) into one at the bottom of the well was observed.

### <Comparative Example 5>

Photosensitive polyvinyl alcohol was obtained according to Synthesis Example 5 and Example 3 of JP 2003-292477 A. The obtained photosensitive polyvinyl alcohol was dissolved in ethanol so as to have a concentration of 5 mg/g to prepare a composition for forming a coating film.

Note that, as polyvinyl alcohol, GOHSENOL EG-30P (manufactured by Mitsubishi Chemical Corporation, saponification degree: 86.3 to 69.0) was used.

The obtained composition for forming a coating film was operated in the same manner as that of Example 1 to form a coating film on an HMDS-treated silicon wafer and to produce a coating plate for cell culture.

A residual film rate was determined in the same manner as that of Example 1.

A cell adhesion test was performed in the same manner as that of Example 1.

The results were shown in Table 1-2.

### <Comparative Example 6>

A coating film was formed on an HMDS-treated silicon wafer and a coating plate for cell culture was produced in the same manner as those of Comparative Example 5, except that when the coating film was formed on the HMDS-treated silicon wafer and the coating plate for cell culture was produced in the Comparative Example 5, after the drying step, exposure was performed for 5 seconds with an ultrahigh pressure mercury lamp (ultraviolet illuminance 20 mW/cm²: UT-150 (illuminance meter manufactured by Ushio Inc.)).

A residual film rate was determined in the same manner as that of Example 1.

A cell adhesion test was performed in the same manner as that of Example 1.

The results were shown in Table 1-2.

### <Comparative Example 7>

A spheroid formation test was performed in the same manner as that of Example 31, except that the composition for forming a coating film was not used in Example 31. The results are shown in Table 5.

A photograph of a microscopic observation result of the spheroid formation experiment of the human induced pluripotent stem cells using mTeSR (trademark) 1 in the well without the composition for forming a coating film was illustrated in Fig. 7.

### <Comparative Example 8>

A spheroid formation test was performed in the same manner as that of Example 35, except that the composition for forming a coating film was not used in Example 35. The results are shown in Table 5.

### <Comparative Example 9>

A spheroid formation test was performed using a 96-well U-bottom cell ultra-low adhesion plate (manufactured by Corning Inc., #4520, volume: 0.3 mL, formed of polystyrene) as a positive control. The results are shown in Table 5.

**[Table 1-1]**

| | Polymerization degree | Saponification degree [mol%] | Solvent composition (mass ratio) | γ-Ray irradiation | Film thickness after coating [nm] | Film thickness after PBS washing [nm] | Residual film rate [%] | Cell adhesion suppression effect |
|---|---|---|---|---|---|---|---|---|
| Example 1 | 250 | 35.8 | Water/EtOH = 3/7 | Not performed | 53 | 32 | 60 | ○ |
| Example 2 | 400 | 37.8 | Water/EtOH = 3/7 | Not performed | 52 | 39 | 73 | ○ |
| Example 3 | 580 | 20.0 | Water/EtOH/PGME = 10/85/5 | Not performed | 50 | 47 | 94 | ○ |
| Example 4 | 580 | 31.6 | Water/EtOH/PGME = 10/85/5 | Not performed | 50 | 46 | 91 | ○ |
| Example 5 | 580 | 42.8 | Water/EtOH/PGME = 10/85/5 | Not performed | 50 | 40 | 80 | ○ |
| Example 6 | 580 | 49.9 | Water/EtOH/PGME = 20/75/5 | Not performed | 53 | 31 | 60 | ○ |
| Example 7 | 1080 | 36.2 | Water/EtOH/PGME = 10/85/5 | Not performed | 53 | 48 | 91 | ○ |
| Example 8 | 1460 | 22.7 | Water/EtOH = 3/7 | Not performed | 44 | 43 | 98 | ○ |
| Example 9 | 1460 | 22.7 | Water/EtOH = 5/95 | Not performed | 51 | 50 | 98 | ○ |
| Example 10 | 1460 | 22.7 | EtOH/PGME = 85/15 | Not performed | 50 | 50 | 98 | ○ |
| Example 11 | 1460 | 22.7 | EtOH/PGME = 70/30 | Not performed | 55 | 53 | 97 | ○ |
| Example 12 | 1480 | 22.7 | EtOH/PGME = 40/60 | Not performed | 49 | 48 | 97 | ○ |
| Example 13 | 1460 | 22.7 | Water/EtOH/PGME = 10/85/5 | Not performed | 52 | 51 | 98 | ○ |
| Example 14 | 580 | 20.0 | Water/EtOH/PGME = 10/85/5 | Performed | 50 | 47 | 94 | ○ |
| Example 15 | 580 | 31.6 | Water/EtOH/PGME = 10/85/5 | Performed | 50 | 44 | 87 | ○ |
| Example 16 | 580 | 42.8 | Water/EtOH/PGME = 10/85/5 | Performed | 51 | 38 | 75 | ○ |
| Example 17 | 580 | 49.9 | Water/EtOH/PGME = 20/75/5 | Performed | 54 | 30 | 56 | ○ |

**Table 1-2]**

| | Polymerization degree | Saponification degree [mol%] | Solvent composition (mass ratio) | γ-Ray irradiation | Film thickness after coating [nm] | Film thickness after PBS washing [nm] | Residual film rate [%] | Cell adhesion suppression effect |
|---|---|---|---|---|---|---|---|---|
| Example 18 | 1080 | 36.2 | Water/EtOH/PGME = 10/85/5 | Performed | 53 | 46 | 87 | ○ |
| Example 19 | 1340 | 29.0 | Water/EtOH/PGME =5/90/5 | Performed | 50 | 47 | 94 | ○ |
| Example 20 | 1460 | 22.7 | Water/EtOH = 5/95 | Performed | 56 | 53 | 94 | ○ |
| Example 21 | 1460 | 22.7 | EtOH/PGME = 85/15 | Performed | 49 | 47 | 97 | ○ |
| Example 22 | 1460 | 22.7 | EtOH/PGME = 70/30 | Performed | 49 | 46 | 93 | ○ |
| Example 23 | 1460 | 22.7 | Water/EtOH/PGME = 10/85/5 | Performed | 55 | 51 | 93 | ○ |
| Example 24 | 1460 | 22.7 | Water/EtOH/PGME = 5/90/5 | Performed | 49 | 48 | 97 | ○ |
| Example 25 | 1560 | 26.3 | Water/EtOH/PGME = 5/90/5 | Performed | 53 | 50 | 95 | ○ |
| Example 26 | 1560 | 31.3 | Water/EtOH/PGME = 5/90/5 | Performed | 53 | 50 | 93 | ○ |
| Example 27 | 1560 | 36.6 | Water/EtOH/PGME = 10/85/5 | Performed | 55 | 51 | 93 | ○ |
| Comparative Example 1 | 230 | 65.4 | Water/EtOH = 3/7 | Not performed | 50 | 4 | 8 | ○ |
| Comparative Example 2 | 360 | 65.7 | Water/EtOH = 3/7 | Not performed | 58 | 3 | 6 | ○ |
| Comparative Example 3 | 200 | 0.0 | Water/EtOH = 3/7 | Not performed | 53 | 43 | 82 | × |
| Comparative Example 4 | 230 | 9. 9 | Water/EtOH = 3/7 | Not performed | 44 | 42 | 97 | X |
| Comparative Example 5 | - | | | Not performed | 20 | 6 | 28 | × |
| Comparative Example 6 | - | | | Not performed | 20 | 17 | 85 | ○ |

**[Table 2]**

| | Absorbance (450 nm - 650 nm) | Protein adsorption rate [%] |
|---|---|---|
| Example 1 | 0.01328 | 12 |
| Example 2 | 0.01264 | 12 |
| Example 3 | 0.01492 | 14 |
| Example 4 | 0.01306 | 12 |
| Example 5 | 0.01384 | 13 |
| Example 6 | 0.01248 | 11 |
| Example 7 | 0.01416 | 13 |
| Example 8 | 0.01748 | 16 |
| Example 9 | 0.01956 | 18 |
| Example 10 | 0.02548 | 23 |
| Example 11 | 0.02946 | 27 |
| Example 12 | 0.02116 | 19 |
| Example 13 | 0.01778 | 16 |
| Example 14 | 0.04054 | 37 |
| Example 15 | 0.01752 | 16 |
| Example 16 | 0.01538 | 14 |
| Example 17 | 0.01428 | 13 |
| Example 18 | 0.0233 | 21 |
| Example 19 | 0.02004 | 18 |
| Example 20 | 0.07894 | 73 |
| Example 21 | 0.05982 | 55 |
| Example 22 | 0.02694 | 25 |
| Example 23 | 0.02866 | 26 |
| Example 24 | 0.01876 | 17 |
| Example 25 | 0.01974 | 18 |
| Example 26 | 0.01458 | 13 |
| Example 27 | 0.01368 | 13 |
| Comparative Example 1 | 0.01194 | 11 |
| Comparative Example 2 | 0.0124 | 11 |
| Comparative Example 3 | 0.44882 | 412 |
| Comparative Example 4 | 0.01712 | 16 |
| Without coating agent | 0.10884 | 100 |

**[Table 3]**

| | Cell adhesion to base film forming agent 2 hours after seeding | Cell aggregate formation one day after seeding |
|---|---|---|
| Example 3 | ○ | ○ |
| Example 4 | ○ | ○ |
| Example 7 | ○ | ○ |
| Example 10 | ○ | ○ |
| Example 11 | ○ | ○ |
| Example 12 | ○ | ○ |
| Example 13 | ○ | ○ |
| Example 14 | ○ | ○ |
| Example 15 | ○ | ○ |
| Example 18 | ○ | ○ |
| Example 19 | ○ | ○ |
| Example 20 | ○ | ○ |
| Example 21 | ○ | ○ |
| Example 22 | ○ | ○ |
| Example 23 | ○ | ○ |
| Example 24 | ○ | ○ |
| Example 25 | ○ | ○ |
| Example 26 | ○ | ○ |
| Example 27 | ○ | ○ |

**[Table 4]**

| | Cell adhesion suppression effect | Cell adhesion to base film forming agent 2 hours after seeding | Cell aggregate formation one day after seeding |
|---|---|---|---|
| Example 28 | ○ | ○ | ○ |
| Example 29 | ○ | ○ | ○ |
| Example 30 | ○ | ○ | ○ |

**[Table 5]**

| | Composition for forming coating film | γ-Ray irradiation | Spheroid formation | | | |
|---|---|---|---|---|---|---|
| | | | iPS cells | | ADSC | |
| | | | mTeSR1 | StemFit | Low serum | Serum-free |
| Example 31 | Example 11 | Not performed | ○ | ○ | ○ | ○ |
| Example 32 | Example 10 | | ○ | ○ | ○ | ○ |
| Example 33 | Example 11 | | ○ | ○ | ○ | ○ |
| Example 34 | Example 10 | | ○ | ○ | ○ | ○ |
| Comparative Example 7 | Absence | | × | × | × | ○ |
| Example 35 | Example 11 | Performed | ○ | ○ | ○ | ○ |
| Example 36 | Example 10 | | ○ | ○ | ○ | ○ |
| Example 37 | Example 11 | | ○ | ○ | ○ | ○ |
| Example 38 | Example 10 | | ○ | ○ | ○ | ○ |
| Comparative Example 8 | Absence | | × | × | × | ○ |
| Comparative Example 9 | - | | ○ | × | ○ | ○ |

In Table 1-1 and Table 1-2, EtOH indicates ethanol, and PGME indicates propylene glycol monomethyl ether.

The saponification degree of polyvinyl acetate in each of Examples 1 to 27 and Comparative Examples 1 to 4 corresponds to a molar ratio (A : B) of the repeating unit (A) to the repeating unit (B).

For example, the molar ratio (A : B) of polyvinyl acetate having a saponification degree of 35.8 mol% is 64.2 : 35.8.

In addition, for example, the molar ratio (A : B) of polyvinyl acetate having a saponification degree of 65.4 mol% is 34.6 : 65.4.

The polyvinyl acetate used in each of Examples 1 to 27 and Comparative Examples 3 and 4 was water-insoluble.

The polyvinyl acetates used in Comparative Examples 1 and 2 were all water-soluble.

The photosensitive polyvinyl alcohol used in Comparative Example 5 was water-soluble.

The coating film obtained using the composition for forming a coating film of each of Examples 1 to 27 had a high residual film rate even after washing with PBS as compared with the coating film obtained using the composition for forming a coating film each of Comparative Examples 1, 2, and 5.

In the coating films obtained using the compositions for forming a coating film of Examples 1 to 27, adhesion and spread of the cells were not observed, and formation of cell aggregates (spheroids) was observed in the well. On the other hand, in the coating films obtained using the compositions for forming a coating film of Comparative Examples 3, 4, and 5, the cells adhered and spread to the bottom surface of the plate. In addition, in order for the coating film obtained using the composition for forming a coating film of Comparative Example 5 to have a residual film rate and a cell adhesion inhibition effect similar to those of Examples, ultraviolet irradiation was required.

The coating films obtained using the compositions for forming a coating film of Examples 1 to 27 had a lower protein adsorption rate than those of Comparative Example 3 and the substrate without a coating film.

In the plate for cell aggregate formation obtained by forming the cell culture base film on the coating film obtained using the composition for forming a coating film of each of Examples 3, 4, 7, 10 to 15, and 18 to 27, the cells selectively adhered to the cell culture base film 2 hours after seeding, and after one day, the cells were detached, and cell adhesion aggregates were formed.

In the coating films obtained using the compositions for forming a coating film of Examples 28 to 30 by the inkjet coating, adhesion and spread of the cells were not observed, and formation of cell aggregates (spheroids) was observed in the well. In addition, in the plate for cell aggregate formation obtained by forming the cell culture base film on the coating film obtained using the composition for forming a coating film, the cells selectively adhered to the cell culture base film 2 hours after seeding, and after one day, the cells were detached, and cell adhesion aggregates were formed.

In the coating plates for cell culture obtained using the compositions for forming a coating film of Examples 31 to 38, only adhesion of the cells was not observed, and formation of cell aggregates (spheroids) into one at the bottom of the well was observed.

## Claims

1. A coating film which is obtained by applying a composition for forming a coating film, the composition containing a copolymer, and which is used to suppress adhesion of a biological substance,
wherein the copolymer is water-insoluble,
the copolymer contains a repeating unit (A) represented by the following Formula (A) and a repeating unit (B) represented by the following Formula (B), and
a molar ratio (A : B) of the repeating unit (A) to the repeating unit (B) in the copolymer is 89 : 11 to 50 : 50, 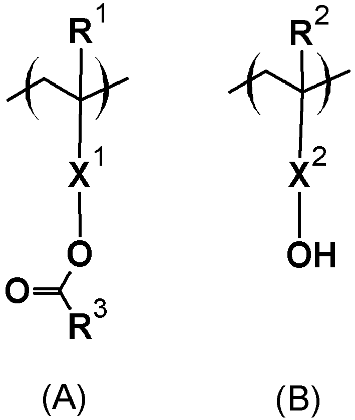
(in the formula, R¹ to R³ each independently represent a hydrogen atom or an alkyl group having 1 to 5 carbon atoms, and X¹ and X² each independently represent a single bond, an ester bond, an ether bond, an amide bond, or an alkylene group having 1 to 5 carbon atoms which may be interrupted by an oxygen atom).

2. The coating film according to claim 1, wherein R¹ and R² are hydrogen atoms, R³ is a methyl group, and X¹ and X² are single bonds.

3. The coating film according to claim 1, wherein a viscosity-average polymerization degree of the copolymer is 200 to 3,000.

4. The coating film according to claim 1, wherein a total mol% of the repeating unit (A) and the repeating unit (B) in all the repeating units in the copolymer is 99.5 mol% or more.

5. The coating film according to claim 1, wherein the copolymer does not contain an azide group.

6. The coating film according to claim 1, wherein the copolymer is not crosslinked in the coating film.

7. A composition for forming a coating film which is used to suppress adhesion of a biological substance, the composition comprising a copolymer,
wherein the copolymer is water-insoluble,
the copolymer contains a repeating unit (A) represented by the following Formula (A) and a repeating unit (B) represented by the following Formula (B), and
a molar ratio (A : B) of the repeating unit (A) to the repeating unit (B) in the copolymer is 89 : 11 to 50 : 50, 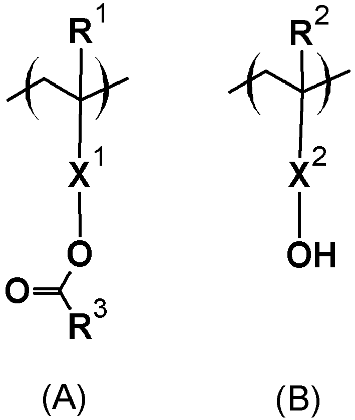
(in the formula, R¹ to R³ each independently represent a hydrogen atom or an alkyl group having 1 to 5 carbon atoms, and X¹ and X² each independently represent a single bond, an ester bond, an ether bond, an amide bond, or an alkylene group having 1 to 5 carbon atoms which may be interrupted by an oxygen atom).

8. The composition for forming a coating film according to claim 7, wherein R¹ and R² are hydrogen atoms, R³ is a methyl group, and X¹ and X² are single bonds.

9. The composition for forming a coating film according to claim 7, wherein a viscosity-average polymerization degree of the copolymer is 200 to 3,000.

10. The composition for forming a coating film according to claim 7, wherein a total mol% of the repeating unit (A) and the repeating unit (B) in all the repeating units in the copolymer is 99.5 mol% or more.

11. The composition for forming a coating film according to claim 7, wherein the copolymer does not contain an azide group.

12. The composition for forming a coating film according to claim 7, further comprising a solvent.

13. The composition for forming a coating film according to claim 12, wherein the solvent contains an alcohol.

14. A coating film which is obtained by applying the composition for forming a coating film according to claim 7 and is used to suppress adhesion of a biological substance.

15. The coating film according to claim 14, wherein the copolymer is not crosslinked in the coating film.

16. The coating film according to claim 1, wherein a thickness of the coating film is 1 to 2,000 nm.

17. A substrate with a coating film, comprising a substrate, and the coating film according to any one of claims 1 to 6 and 14 to 16 disposed on the substrate.

18. A cell culture vessel comprising the substrate with a coating film according to claim 17.

19. A method for manufacturing a coating film, the method comprising the steps of:
supplying the composition for forming a coating film according to any one of claims 7 to 13 onto a substrate; and
drying the composition for forming a coating film supplied onto the substrate.

20. The method for manufacturing a coating film according to claim 19, wherein the copolymer is not crosslinked.
